# EUROPEAN PATENT APPLICATION

(11) **EP 2 141 236 A1**
(43) Date of publication of application: **06.01.2010**
(21) Application number: 08012016.5
(22) Date of filing: 03.07.2008
(51) Int. Cl.: C12N 15/52, A61K 31/365, C12P 17/02, C07D 305/12

(54) **Process for production of lipstatin and microorganisms therefore**

(71) Applicant: KRKA, D.D., Novo Mesto, 8501 Novo mesto (SI)
(72) Inventor: Fujs, Stefan, 9201 Puconci (SI); Kuscer, Enej, 1000 Ljubljana (SI); Petrovic, Hrvoje, 1000 Ljubljana (SI); Sladic, Gordan, 8000 Novo mesto (SI); Gasparic, Ales, 8000 Novo mesto (SI)
(74) Representative: HOFFMANN EITLE

(57) **Abstract**

This invention relates to improved processes for the production of lipstatin and /or orlistat and means therefore. In particular, the invention relates to microorganisms characterized by abolished or reduced activity of the BKD complex, e.g. mutated branched-chain 2-oxo acid dehydrogenase (BKD) having reduced or abolished activity, nucleotide sequences suitable for achieving reduced or abolished BKD activity, and microorganisms having reduced or abolished BKD activity. The invention in particular relates to lipstatin-producing microorganisms of the genus Streptomyces. Furthermore, this invention relates to the use of these nucleotide sequences, microorganisms or BKD having reduced or abolished activity in the production of lipstatin and/or orlistat.

## Description

### Field of the invention

This invention relates to mutated or recombinant microorganisms and their use in the production of lipstatin and/or orlistat. More specifically this invention relates to microorganisms having a reduced or abolished activity of at least one functional subunit of the branched-chain 2-oxo acid dehydrogenase (BKD) complex, in particular branched-chain 2-oxo acid dehydrogenase (BKD) having reduced or abolished activity, nucleotide sequences suitable for achieving reduced or abolished activity of the BKD complex, specifically BKD, and microorganisms having reduced or abolished activity of the BKD complex, specifically BKD. The invention also encompasses sequences encoding the E1 alpha subunit of a microorganism of the genus Streptomyces. The invention in particular relates to microorganisms capable of producing lipstatin, specifically Actinomycetes, more specifically of the genus Streptomyces. Furthermore, this invention relates to the use of these nucleotide sequences, microorganisms or BKD complex/BKD having reduced or abolished activity in the production of lipstatin and/or orlistat.

### Background of the invention

Lipstatin is known as an intermediate of orlistat, which is an agent inhibiting the activity of pancreatic lipase. Orlistat is known in the art for the preparation of medicaments for preventing and treating hyperlipaemia and obesity.

Lipstatin is the key intermediate for the production of tetrahydrolipstatin (THL, Orlistat), a lipase inhibitor useful for treatment of diseases associated with obesity. Although lipstatin (orlistat) can be synthesized chemically, fermentation processes to produce lipstatin are more economical. Weibel et al. (1987 J Antibiot, 40(8):1081-5) disclose generally the fermentation process for lipstatin and E. Hochuli et al. (1987 J Antibiot (Tokyo);40(8):1086-91.) disclose the structural chemistry for lipstatin.

US 4,598,089 and EP 129,748 disclose the cultivation and fermentation of particular S. toxytricini strain (i.e., Streptomyces toxytricini NRRL15443) producing lipstatin. Patent applications Teva (WO 03/048335), Biotika (WO 2007/078263), Biocon (WO 2004/003212) and Krka (WO 2007/134836), as well as the early Roche patent application US 4,598,089 and EP 129 748, further disclose various fermentation processes for lipstatin production. All are based on a fermentation process using natural triglycerides or fatty acids, particularly linoleic and caprylic acids, as a main source of carbon and source of fatty acid precursors used for lipstatin biosynthesis. Optionally, feeding of L-leucine or N-formyl-L-leucine during the fermentation process is disclosed (US 4,598,089 and EP 129 748) and is identified to be particularly useful in order to increase the final yield of lipstatin.

Known processes of fermentative production of lipstatin are economically attractive but have the disadvantage of a low purity of lipstatin. It is the aim of the present invention to provide microorganisms, nucleotide sequences and processes for the improved fermentative production of lipstatin. In particular it is an aim of the present invention to provide microorganisms, nucleotide sequences and processes for fermentative production of lipstatin having improved purity.

### Summary of the invention

The present invention relates to an improved process for the production of lipstatin and/or orlistat, and means therefore. It is based on the unexpected finding that the branched-chain 2-oxo acid dehydrogenase (BKD) complex contributes to the formation of branched chain analogues of lipstatin. Surprisingly, the reduction or abolishment of BKD complex activity leads to an improved process for production of lipstatin and/or orlistat, characterized by a reduced or abolished content of branched chain lipstatin and/or orlistat analogues. Thus, the present invention significantly reduces the disadvantages of known processes of fermentative lipstatin/orlistat production by employing means to reduce or abolish BKD complex activity.

Specifically, the present invention relates to:
A microorganism capable of producing lipstatin, characterized by being unable to grow on a minimal medium comprising valine, leucine and isoleucine as sole carbon source, preferably characterized by a reduced or abolished activity of at least one functional subunit of the branched-chain 2-oxo acid dehydrogenase multi-enzyme complex. More specifically, the invention relates to a microorganism wherein the activity of at least one gene coding for the E1 alpha, E1 beta, E2 and E3 subunits of the branched-chain 2-oxo acid dehydrogenase multi-enzyme complex or one or more regulatory sequences promoting the activity of one or more of these genes has been reduced or abolished,
   preferably wherein the activity of the E1 alpha gene coding for branched-chain 2-oxo acid dehydrogenase and/or of a regulatory sequence promoting activity thereof has been reduced or abolished.

In one specific embodiment, the microorganism of the invention is characterized by a specific activity of branched-chain 2-oxo acid dehydrogenase below 1.5 U/mg total protein, preferably 0 U/mg total protein, as determined in a cell free extract, wherein 1 U represents the generation of 1 nmol NADH per minute. Enzyme activity can be determined as defined herein. Further guidance is given in the art, e.g. Marshall and Sokatch, J. Bact. 110. 1073-1081, 1972.

The microorganism of the invention can belong to Actinomycetes, preferably belongs to the genus. Streptomyces, and more preferably is selected from the Streptomyces lavendulae group consisting of Streptomyces toxytricini and Streptomyces virginiae.

The invention encompasses a nucleotide sequence suitable for reducing or abolishing, in a microorganism capable of producing lipstatin, the activity of at least one functional subunit of branched-chain 2-oxo acid dehydrogenase multi-enzyme complex,
preferably suitable for reducing or abolishing the specific activity of branched-chain 2-oxo acid dehydrogenase to less than 1.5 U/mg total protein as determined in a cell free extract, wherein 1 U represents the generation of 1 nmol NADH per minute,
preferably selected from a mutated nucleotide sequence derived from a sequence coding for branched-chain 2-oxo acid dehydrogenase, and a mutated promoter sequence derived from a promoter of branched-chain 2-oxo acid dehydrogenase.

Also provided by the invention is a nucleotide sequence encoding the E1 alpha subunit of the branched-chain 2-oxo acid dehydrogenase of an organism belonging to the genus Streptomyces, comprising the sequence according to SEQ ID NO: 1, as well as variants thereof, comprising
a) one or more nucleotide additions, deletions, substitutions or inversions;
b) the sequence according to a) having at least 80% identity, preferably at least 90% identity with SEQ ID NO: 1;
c) a nucleic acid sequence capable of hybridizing under stringent conditions to the a nucleotide sequence, or its complement, according to a) or b), or to SEQ ID NO: 1;
wherein said sequence according to a), b), or c) has the capacity to encode the E1 alpha subunit of the branched-chain 2-oxo acid dehydrogenase of an organism belonging to the genus Streptomyces.

The invention pertains to a process for obtaining a microorganism as defined above, comprising mutating a microorganism and selecting a mutant having decreased or abolished activity of at least one gene coding for the four subunits of the branched-chain 2-oxo acid dehydrogenase multi-enzyme complex, e.g. comprising mutating a nucleotide sequence coding for one or more subunits of the BKD multi-enzyme complex or their regulatory sequences as defined herein, or mutating a sequence encoding the E1 alpha subunit of the BKD complex or its variants as defined herein.

The invention also relates to a process for the manufacture of lipstatin comprising use of the microorganism as defined herein, and microorganisms obtainable by the methods of the present invention, e.g. using the nucleotide sequences of the present invention.

The invention also relates to a process for the manufacture of orlistat, comprising manufacture of lipstatin as defined above, and hydrogenation of lipstatin to obtain orlistat.

The microorganisms defined herein, including microorganisms obtainable by the methods of the present invention, or a nucleotide sequence according to the invention can be used in a process for producing lipstatin and/or orlistat. The invention also extends to Lipstatin and/or Orlistat obtainable by the methods of the present invention, preferably characterized by a content of branched chain analogues below 5% by weight.

Finally, the invention relates to a process for the manufacture of a medicament comprising orlistat, comprising use of a microorganism according to the invention, a nucleotide sequence according to the invention, lipstatin according to the invention or orlistat according to the invention.

### Brief description of the figures

Figure 1: Biosynthetic precursors of lipstatin
Figure 2: Sequence information on the E1 alpha subunit of the BKD multi-enzyme complex

### Detailed description of the invention

### 1. Biosynthesis of lipstatin and branched analogues

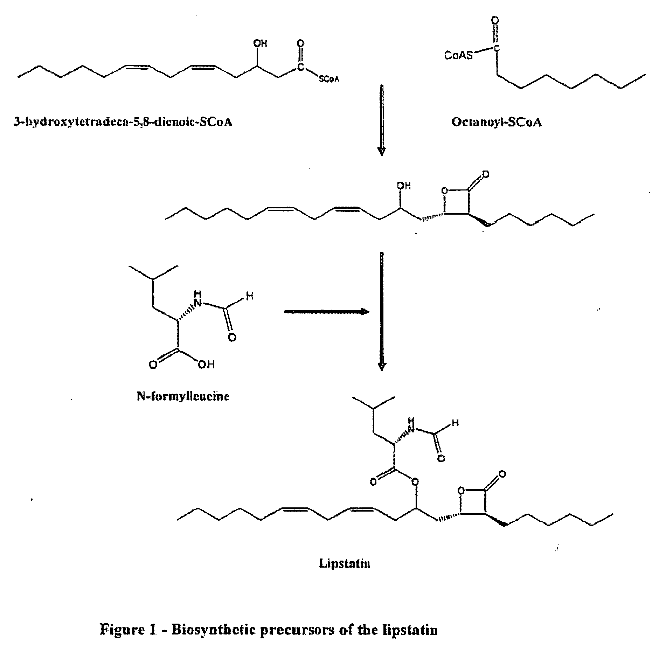

The biosynthesis of lipstatin by Streptomyces toxytricini (S. toxytricini) was studied using various approaches including isotope labelling experiments. It has been demonstrated that lipstatin is biosynthesized in S. toxytricini via condensation of a C-14 and C-8 precursor (Fig 1). The β - lactone moiety, which is crucial for lipase inhibitory activity, is formed by a Claisen condensation of the C14 carboxylic acid or its thioester with octanoyl-CoA. The formyl leucine moiety is then attached by a putative amino-acylase on the hydroxyl group located on the lipophilic C22 precursor of lipstatin. Schuhr et al., (2002 J Org Chem. 67(7):2257-62.) have demonstrated that the addition of the formyl group is likely attached to the leucine after the aminoacylation of the lipophilic C22 precursor of lipstatin (Fig 1) .

It is known that in the process of lipstatin biosynthesis a number of undesired analogues are produced (e.g. Eisenreich et al. 2003 (J Med Chem. 46(19):4209-12)).

The present inventors have found that the branched-chain 2-oxo acid dehydrogenase complex contributes to the formation of branched chain lipstatin analogues which can significantly contribute to undesired impurities present in the broth at the end of the fermentation process. It is hence the teaching of the present invention to reduce or abolish the activity of this complex, e.g. the activity of branched-chain 2-oxo acid dehydrogenase, in order to obtain lipstatin comprising a reduced amount of undesired analogues.

### 2. Branched-chain 2-oxo acid dehydrogenase (BKD) complex

In the context of the present invention, the term "branched-chain 2-oxo acid dehydrogenase (bkd) gene complex" means a multi-enzyme complex composed of four functional subunits: E1-α (branched-chain 2-oxo acid dehydrogenase), E1-β (decarboxylase function), E2 subunit (containing a dihydrolipoamide acyltransferase function) and E3 (containing a dihydrolipoamide dehydrogenase function). The BKD complex catalyzes the oxidative decarboxylation of 2-oxo isovalerate, 2-oxo 2-methylvalerate, and 2-oxo isocaproate, releasing CO2 and generating the corresponding acyl-CoA analogues and NADH (Denoya et al., J. Bact., 177, 3504-3511, 1995). Actinomycetes contain two sets of clustered genes, each of which encodes E1-α, E1-β and E2 subunits (i.e. in *S. coelicolor bkdA1B1C1* and *bkdA2B2C2*). In Streptomyces the E3 subunit is not linked to the gene cluster and likely resides elsewhere in the genome (Sprusansky et al., J. Bact. 187, 664-671, 2005).

In the context of the present invention, the term "branched-chain 2-oxo acid dehydrogenase", short "BKD", comprises the E1-α and E1-β subunits, which are responsible for branched-chain 2-oxo acids dehydrogenase and decarboxylase activity (EC 1.2.4.4), and more specifically relates to the E1-α subunit.

One specific example of BKD is encoded by a gene for the E1-α subunit comprising the nucleotide sequence according to SEQ ID NO: 1 which shares similarity to the sequence of the gene bkdF (branched-chain 2-oxo acid dehydrogenase E1 alpha subunit) from Streptomyces avermitilis MA-4680 (accession NP_825553, version GI:29830919), see also WO 95/04150. This reference, however, relates to a microorganism (Streptomyces avermitilis), which is not capable of producing lipstatin. Hence, this reference pertains to an entirely different metabolic pathway, and provides the skilled person with no guidance of relevance for the present invention.

The present invention is not, however, limited to a specific example of BKD. It encompasses all enzymes, and genes coding therefore, which have BKD activity in the microorganisms as defined herein.

Thus, the present invention comprises the manipulation/mutation of any one of the above genes encompassed by the BKD complex. As a specific, non-limiting example, the following disclosure centres on the E1α subunit. This disclosure, however, equally applies to the E1β, E2 and E3 subunits of the BKD complex as defined above. The present invention also encompasses the manipulation/mutation of two, three or all of the genes of the complex.

### 3. Microorganisms of the invention

In the present application, the designation of microorganisms follows common general practice, as reflected e.g. in "Bergey's Manual of Determinative Bacteriology".

This invention relates to microorganisms capable of fermentative production of lipstatin, characterized by the inability to grow on a minimal medium comprising one or more selected from valine, leucine or isoleucine as the sole carbon source. Microorganisms of the invention in which the activity of at least one member of the bkd complex, e.g. the E1 alpha subunit, is reduced or abolished will be unable to grow on the said minimal medium. In the context of the present invention, "unable to grow" means that growth is significantly reduced as compared to a suitable control, e.g. reduced by at least 50%, 70%, 80%, 90% at a given time, or is completely absent. The skilled person will select a suitable control strain on the basis of general scientific principles. Growth will be compared at a suitable time-point, when growth of the control strain is unimpeded e.g. by depletion of nutrients or other factors inhibiting growth of the control. Moreover, the skilled person knows various adequate means to determine growth. For example, Streptomyces mycelium can be directly quantified after harvesting, e.g. by determining of dry or wet weight, growth can be established on the basis of e.g. tritiated thymidine incorporation, or on the basis of fluorescent staining of nuclei, coupled with appropriate quantification methods (e.g. scintillation, fluorescence). Preferably there is no growth at all.

Moreover, the microorganism is characterized by a reduced or abolished activity of the BKD complex, in particular of BKD. The skilled person knows procedures to determine whether a given microorganism is capable of production of lipstatin. Moreover, the experimental section of this application can be taken as additional guidance on suitable procedures.

In one embodiment, the microorganism belongs to Actinomycetes, preferably belongs to the genus Streptomyces, and in particular is selected from Streptomyces toxytricini or Streptomyces virginiae. Whenever the present application specifically refers to Streptomyces toxytricini, the disclosure equally comprises Streptomyces virginiae. Where the present application refers to "microorganism capable of producing lipstatin", S. toxytricini and S. virginiae are encompassed as specific examples. Moreover, it is also considered in the context of the present invention to utilize other microorganisms, including genetically modified microorganisms, provided they have the ability to fermentatively produce lipstatin.

The invention relates to methods of generating said microorganism, e.g. Streptomyces, in particular S. toxytricini strains, and the use of the strains in the fermentation process for the production of lipstatin. The reduction or complete inhibition of the activity of the BKD complex, in particular branched-chain 2-oxo acid dehydrogenase activity (BKD activity) in S. toxytricini strains results in significant reduction of branched-chain lipstatin-related analogues during the fermentation process.

Procedures to generate Streptomyces toxytricini for further manipulation according to the present invention are known in the art. Moreover, suitable strains of Streptomyces toxytricini are commercially available. One specific, non-limiting example of a suitable strain is Streptomyces toxytricini having the deposit number CBS 566.68. Further examples of embodiments of the present invention comprise Streptomyces toxytricini A676 and A2072 deposited as CBS122912 and CBS122981, respectively, both deposited on June 5, 2008, at the Centraalbureau voor Schimmelcultures, Uppsalalaan 8, 3584 CT Utrecht, The Netherlands.

The invention relates to producers of lipstatin, like strains of Streptomyces toxytricini, in which the branched-chain 2-oxo acid dehydrogenase complex activity (BKD complex activity), in particular the BKD activity, is reduced or abolished. Such microorganisms show the surprising technical effect that the production of branched chain lipstatin analogues is reduced.

In the context of the present invention, "reduced or abolished" BKD activity means that the enzymatic activity is lowered as compared to a reference value. A suitable reference value is the enzymatic activity determined in a reference strain of Streptomyces toxytricini (control), wherein a reference strain is the microorganism prior to the manipulation/mutation to reduce or abolish BKD according to the present invention. In other words, "reduced or abolished" BKD activity is determined by comparing the same microorganism before (control) and after the manipulations to reduce or abolish BKD activity (embodiment of the invention). A suitable reference strain would e.g. comprise a wild type bkd gene and/or regulatory sequences. Any of the specific strains of Streptomyces toxytricini defined in the present application, which do not represent embodiments of the invention, can serve as a reference strain for manipulations in the respective strain. The comparison can involve purified or partially purified enzyme, or be based on complete or disrupted microorganisms.

The skilled person knows suitable methods to determine the activity of all individual subunits of the BKD complex. For example, the skilled person knows suitable procedures to determine BKD activity as described in Marshall and Sokatch, J. Bact. 110. 1073-1081, 1972. Moreover, the experimental section of this application provides a particular non-limiting example of a suitable procedure to determine BKD activity.

To determine BKD activity in the context of this invention, the skilled person can obtain a cell free extract by disruption of cells by suitable means and removal of particulate matter (cell debris). For example, the skilled person can lyse cells by suitable means, such as chemicals, including detergents, solvents, suitable hypoosmotic conditions, or physical means, like grinding, freeze-thawing or sonication, or any combinations thereof. A specific example is sonication of cells, e.g. four times for 15 seconds at 100% amplitude with Labsonic M (Sartorius) with 30 seconds pause between two cycles, in a suitable buffer, e.g. K-phosphate buffer pH 8.0. The procedure can advantageously be performed on ice.

Cell debris can then be removed e.g. by filtration, centrifugation or sedimentation. Specifically, cell debris can be removed by centrifugation at 14000 rpm for 10 minutes. In a specific embodiment, the microorganism of the invention is lysed by sonication and cell debris removed by centrifugation, and the supernatant collected as the cell free extract for determining total protein and BKD activity.

The skilled person will incubate the preparation containing BKD, e.g. the cell free extract, with a suitable substrate of the respective enzyme, comprising e.g. one or more of valine, leucine, isoleucine, 2-oxo isovalerate, 2-oxo 2-methylvalerate, or 2-oxo isocaproate, in the presence of NAD⁺ and determine BKD activity by measurement of the generated NADH.

In other words, the skilled person knows suitable means to prepare a cell free extract from the microorganism in which the activity of the BKD complex and/or BKD is to be measured, and incubate this cell free extract in a reaction mixture comprising e.g. L-valine as the substrate, together with suitable reagents, and determine enzyme activity spectrophotometrically at 340 nm. Suitable reagents comprise NAD+, coenzyme A (CoA), dithiotreitol (DTT), MgCl₂, thiamine pyrophosphate (TPP) in a suitable buffer, such as K-phosphate buffer. A suitable pH can be chosen, e.g. pH 8. Specific conditions and concentrations of the reagents can be chosen in accordance with example 13.

In particular, the term "reduced" BKD activity in the context of this invention means a specific activity of branched-chain 2-oxo acid dehydrogenase below 1.5 U/mg of total protein in the cell free extract as described above, wherein 1 U is defined as 1 nmol NADH produced per minute, preferably below 1.0, 0.5, 0.25, 0.1, or 0.01 U/mg protein. "Abolished" activity means any activity that is below the detection limit of the respective enzymatic assay, and preferably means absent enzymatic activity, i.e. 0 U/mg protein.

Total protein in the cell free extract can be determined by standard procedures, e.g. by commercially available kits, such as a Bradford assay (Bradford, M., Anal. Biochem., 72:248, 1976) .

Typical BKD activities for a reference (wild-type) strain of Streptomyces toxytricini are in the range of about 3.0 U/mg to about 4.0 U/mg of total protein in a cell free extract, as can be ascertained from the experimental section of this application.

Advantageously the activity of the BKD complex, e.g. BKD enzymatic activity, is at least 50% lowered as compared to the reference value (control), preferably at least 70% lowered, more preferably at least 80%, 90% or 95% lowered, most preferably at least 99% lowered. In one embodiment BKD activity is 0% as compared to the reference value, i.e. BKD activity is "abolished". Any value lying below the detection limit of the assay to determine BKD activity will be considered to represent 0% BKD activity, i.e. abolished BKD activity.

### 4. Means and manipulations to reduce or abolish BKD activity

The present invention is not limited with respect to the means and manipulations that can be used to reduce or abolish BKD activity. In the following, specific examples of suitable means and manipulations are provided.

### A) Selection/Isolation of suitable strains

Typically, the present invention will involve the manipulation of Streptomycetae strains producing lipstatin and of known strains of Streptomyces toxytricini in order to reduce or abolish BKD activity. However, it is also contemplated to specifically isolate and select suitable strains, e.g. from natural sources, such as soil, which are characterized by reduced or abolished BKD activity.

### B) Genetic modification

In some embodiments of the present invention, the microorganisms are genetically modified in order to reduce or abolish activity of the BKD complex, e.g. BKD activity.

Specifically, the present invention relates to Streptomyces lipstatin-producing mutants of the BKD complex that lack BKD complex activity, e.g. BKD mutants that lack BKD activity.

Generation of mutants devoid of branched-chain 2-oxo acid dehydrogenase activity has been reported for Bacillus subtilis (Willecke and Pardee, 1971, J. Biol. Chem. 246, 5264-72), Pseudomonas putida (Martin et al., 1973, J. Bacteriol., 115, 198-204) and Streptomyces avermitilis (Hafner et al., 1990, J. Antib., 44, 349-356). None of these publications relates to lipstatin producing microorganisms.

Mutations may involve one or more genes of the BKD complex, as well as their regulatory sequences. For example, mutations may involve the gene coding for branched-chain 2-oxo acid dehydrogenase and/or regulatory sequences supporting BKD activity.

In the context of the invention "gene" is to be understood in its broadest sense, and includes both coding and non-coding sequences. In a preferred embodiment the genetic modification concerns the coding sequence of the gene, e.g. the bkd gene. One example of a bkd gene sequence that can be mutated is the nucleotide sequence of SEQ ID NO: 1 of the branched-chain 2-oxo acid dehydrogenase E1α subunit. In the following, the term "bkd gene" is meant to include the branched-chain 2-oxo acid dehydrogenase E1α subunit, more specifically the fragment of the E1α subunit according to SEQ ID NO: 1, but is not meant to be limited to this particular exemplary embodiment.

In the context of the invention "regulatory sequences" are to be construed broadly. The encompass both cis- and transacting sequences, provided they enhance BKD activity. Specific examples of regulatory sequences comprise sequences enhancing the transcription and/or translation of the branched-chain 2-oxo acid dehydrogenase gene, for example operator sequences, promoter sequences or enhancer sequences, for example sequence enhancing expression of the BKD gene and or enhancing the transcription and/or translation of the branched-chain 2-oxo acid dehydrogenase gene.

Suitable methods of introducing mutations comprise the following:

### a) Random mutagenesis

Suitable microorganisms as defined herein can be exposed to mutagenesis and mutants characterized by reduced or abolished BKD complex activity can be screened for and isolated. The skilled person knows suitable methods of mutagenesis e.g. by means of exposure to mutagenic chemical agents (e.g. N methyl-N-nitro-N-nitrosoguanidine) and/or mutagenic electromagnetic waves (e.g. X-rays and/or UV light); followed by selecting a mutant having decreased or abolished activity of the BKD complex, in particular branched-chain 2-oxo acid dehydrogenase, by e.g. applying specifically designed selective growth media.

### b) Gene disruption/replacement procedure

The invention also encompasses targeted gene disruption of the bkd complex, in particular of the bkd gene. Targeted gene disruption can be achieved e.g. by site directed mutagenesis, and/or gene replacement. A suitable method of gene replacement is homologous recombination. The skilled person is familiar with suitable techniques (Kieser et al., 2000, Practical Streptomyces genetics, A laboratory Manual.ISBNO-7084-0623-8)

For example a mutant sequence of SEQ ID NO: 1 can be prepared, and used for homologous recombination by introducing the mutated sequence into a microorganism as defined herein. The parts of SEQ ID NO: 1 which are not mutated will align with the respective sequences in the genome of the microorganism, and will result in homologous recombination. The skilled person understands that mutated sequences that are suitable for reducing or abolishing BKD activity by homologous recombination have to fulfil two criteria: they must show sufficient homology to provide for homologous recombination, but contain sufficient mutations to disrupt, or reduce, activity of BKD in the resulting recombinant, or transformed microorganism.

### c) Pharmacological inhibition of the BKD complex

The present invention also contemplates inhibition of BKD using a suitable inhibitor. Inhibitors may be small organic molecules, but also encompass peptides and proteins. One specific example of inhibitory proteins are antibodies, or antibody fragments specifically recognizing, and inhibiting, BKD. Means to generate such antibodies are known to the skilled person. The skilled person also knows means for delivery of such antibodies, e.g. the intracellular expression of suitable antibody fragments in the microorganisms of the invention. When a small organic molecule is used as the inhibitor, it is contemplated to add the inhibitor to the culture medium, or to generate the inhibitor from a suitable precursor in situ.

Irrespective of the particular mechanism of inhibiting the BKD complex, this will inevitably result in a microorganism characterized by being unable to grow in a medium comprising only one or more of valine, leucine and isoleucine as the sole carbon source. This phenotypic characteristic serves as a simple and convenient selection means for readily obtaining the microorganisms of the invention.

### C) Nucleotide sequences of the invention

The invention also relates to nucleotide sequences suitable for decreasing or abolishing the activity of the BKD complex in a microorganism, in particular a microorganism belonging to the genus Streptomyces, more particularly to lipstatin-producing Streptomycetae strains. The invention also relates to nucleotide sequences suitable for the genetic modifications described herein. In particular the invention relates to mutated nucleotide sequence coding for one or more of the subunits of the bkd complex, in particular, bkd, having a reduced or abolished activity, or nucleotide sequences suitable for disrupting the native bkd complex, in particular the bkd gene, e.g. bkd derived mutants suitable for gene disruption by homologous recombination, e.g. as discussed above.

A specific example of a nucleotide sequence of the invention is a full or partial bkd sequence having a frame shift mutation, e.g. a frame shift mutation caused by the deletion of one or two nucleotides at an internal restriction site, e.g. the PvuI site, which is suitable for gene disruption by homologous recombination.

A further example of a nucleotide sequence of the present invention is a full or partial bkd sequence having an expression cassette comprising a resistance gene inserted therein, which is suitable for gene disruption by homologous recombination.

Also encompassed are vectors, plasmids and any kinds of recombinant nucleotide sequences comprising the nucleotides sequences of the invention, such as the said mutated bkd sequences, or modified regulatory sequences. Such vectors, plasmids or recombinant nucleotide sequences are useful in the processes of generating the microorganisms of the present invention.

The invention also encompasses antisense nucleotide sequences, and siRNA suitable for reducing or abolishing the activity of BKD.

Suitable genetic modifications comprise mutations, such as additions, deletions, substitutions, or inversions of one, two, three, four or more nucleotides. Mutations may comprise 1 to 500, 1 to 250, 1 to 100, 1 to 50, 1 to 25, 1 to 10 or 1 to 5 nucleotides.

When mutations are introduced into a sequence comprising SEQ ID NO: 1, the mutants may have less than 95, 90, 80, 70, 60, 50, 40, 30, 20, or less than 10% homology with SEQ ID NO: 1, provided they are suitable to reduce or abolish BKD activity, as defined herein.

Nucleotides of the present invention also encompass sequences coding for one or more of the subunits of the bkd complex in a microorganism capable of producing lipstatin, in particular a microorganism as further defined herein. For example, the present invention comprises a nucleotide sequence encoding the E1 alpha subunit of the branched-chain 2-oxo acid dehydrogenase of an organism belonging to the genus Streptomyces, comprising the sequence according to SEQ ID NO: 1.

The invention also encompasses variants of such nucleotide sequences, e.g. DNA sequences or RNA sequences, comprising
a) one or more nucleotide additions, deletions, substitutions or inversions, e.g. 1 to 30, 1 to 20 or 1 to 10 such modifications;
b) the sequence according to a) having at least 80% identity, preferably at least 90%, more preferably at least 95%, or at least 99% identity with a sequence of a), in particular with SEQ ID NO: 1;
c) a nucleic acid sequence capable of hybridizing under stringent conditions to the a nucleotide sequence, or its complement, according to a) or b), or to SEQ ID NO: 1, wherein stringent conditions are defined as commonly known in the art;
wherein said sequence according to a), b), or c) has the capacity to encode one of the subunits of the bkd complex, preferably the E1 alpha subunit of the branched-chain 2-oxo acid dehydrogenase of a microorganism as defined herein, preferably belonging to the genus Streptomyces.

The nucleic acids as defined above include nucleic acid sequences, e.g. DNA sequences or RNA sequences, encoding a partial sequence of the branched-chain 2-oxo acid dehydrogenase of an organism belonging to the genus Streptomyces, said sequence comprising the sequence of SEQ ID NO: 1. In particular, the sequences include sequences which are (a) at least part of the sequence set out in the appended sequence listing; or (b) a variant of a sequence (a) which encodes a polypeptide which is at least 80%, preferably at least 90%, identical with the corresponding peptide as set out in Appendix (SEQ ID NO: 1). In particular, these sequences include such encoding branched-chain 2-oxo acid dehydrogenase (complex) of lipstatin-producing bacteria from genus Streptomyces, or any subunit thereof.

The invention also pertains to an isolated nucleic acid sequence according to the above, further comprising a region that regulates the expression of said branched-chain 2-oxo acid dehydrogenase complex or one or more genes thereof.

The invention also encompasses microorganisms comprising any of the above nucleic acid sequences.

Such nucleotide sequences are useful as a starting material for genetic manipulations, i.e. they represent the starting point for creating the nucleotide sequences capable of reducing or abolishing activity of the bkd complex. Moreover, such sequences find use in identifying additional sequences coding for subunits of the bkd complex in the microorganisms of the invention.

### D) Process of obtaining the microorganism of the invention

In a further aspect, the present invention relates to a process for obtaining a microorganism according to the present invention.

The process may comprise selection of pre-existing microorganisms having reduced or abolished BKD complex activity, or manipulations to reduce and abolish BKD complex activity followed by selection of microorganisms having the desired phenotype. In the context of these manipulations it is contemplated to apply known methods of molecular biology, such as reflected e.g. in the laboratory manual (Sambrook, and Russell, 2000, Molecular Cloning: A Laboratory Manual, ISBN 978-087969577-4). As pointed out above, the growth characteristics in medium comprising one or more of valine, leucine or isoleucine as the sole carbon source can be used as a selection means in the process of creating the microorganisms of the invention.

The process may comprise mutating a microorganism belonging to the genus Streptomyces, e.g. by means of exposure to mutagenic chemical agents (e.g. N methyl-N-nitro-N-nitrosoguanidine) and/or mutagenic electromagnetic waves (e.g. X-rays and/or UV light) and selecting a mutant having decreased or abolished activity of branched-chain 2-oxo acid dehydrogenase

The process according to the present invention may also comprise mutating the gene coding for a subunit of the bkd complex, e.g. branched-chain 2-oxo acid dehydrogenase by site directed mutagenesis and/or homologous recombination with a nucleotide sequence capable of reducing or abolishing activity of branched-chain 2-oxo acid dehydrogenase.

The process for producing a microorganism of the present invention may further comprise using a nucleotide sequence according to the present invention.

To the extent that in the process of producing the microorganism of the present invention a resistance marker has been introduced, the invention comprises the selection of variant microorganisms characterized by the loss of the resistance marker, but retaining reduced or abolished BKD activity. Such microorganism are also referred to as "secondary" mutants.

### E) Process for producing lipstatin and/or orlistat

The present invention also relates to a process for the manufacture of lipstatin and/or comprising use of the microorganism according to the present invention. Said process may in particular comprise fermentation of the microorganism of the present invention in a suitable culture medium, and harvesting lipstatin from said culture. Suitable culture media are known to the skilled person and are exemplified in the experimental section.

The process of the present invention may further comprise hydrogenation of lipstatin to obtain orlistat by procedures and means known to the skilled person.

### F) Uses according to the invention

The invention also encompasses the use of a microorganism or a nucleotide sequence according to the invention in a process for producing lipstatin and/or orlistat.

### G) Lipstatin according to the invention

The proportion of the lipstatin-related impurities (i.e. branched chain lipstatin analogues) at the end of a conventional fermentative process can well exceed 10% of the final lipstatin by weight (Eisenreich et al., 1997 J Biol Chem. 272(2):867-74) thus complicating the downstream processes and compromising the purity of the final product orlistat.

Thus, the invention relates to lipstatin having a low content of branched chain lipstatin analogues by weight (as determined on the basis of total content of lipstatin and branched chain lipstatin analogues), specifically less than 10% by weight, preferably no more than 5% by weight, more preferably no more than 2.5% by weight, and most preferably no more than 1% by weight. In one embodiment of the invention lipstatin is free of branched chain lipstatin analogues. In particular this applies to lipstatin directly obtained from the fermentation broth without purification steps directed at the removal of branched chain analogues, for example lipstatin obtainable by extraction of Streptomyces biomass with acetonitrile.

Specific examples of lipstatin of the present invention include lipstatin comprising branched chain lipstatin analogues in an amount of 0.1% to 5%, 0.1 to 3%, 0.1 to 2%, 0.1 to 1% or 0.1 to 0.5% by weight of total lipstatin, in particular determined in lipstatin directly obtainable from the fermentation broth without further purification steps aimed at removing branched chain analogues, e.g. obtainable by direct acetonitrile extraction of the biomass of a microorganism of the invention isolated from the fermentation broth. A suitable determination method for the analogues is LC-MS.

In particular, the present invention relates to lipstatin comprising less than 1.7%, preferably no more than 0.5% or no more than 0.3% of lipstatin analogues with a molecular mass of 506 g/mol.

Thus, the present invention provides lipstatin at an industry scale and with the desired purity, as well as industry scale production processes for pure lipstatin.

Lipstatin having the purity as defined above is obtainable by the processes described in this application, in particular by fermentation of microorganisms of the present invention. In particular, further purification steps, such as purification steps to remove branched chain lipstatin analogues, are not required in order to obtain the above specified lipstatin.

The branched lipstatin analogues are converted to corresponding orlistat analogues when the lipstatin preparation is hydrogenated to an orlistat preparation. Accordingly, the present invention also provides an orlistat preparation, e.g. obtainable by fermentation, in particular fermentation without further purification steps to remove branched chain analogues, having the low levels of orlistat analogues as defined above in the context of lipstatin.

Finally, the present invention relates to the manufacture of a pharmaceutical preparation containing orlistat as defined herein, in particular orlistat obtainable by fermentative production of lipstatin by use of a microorganism, nucleotide, or process according to the invention.

### Experiments

The following are detailed examples of the experimental procedures used to clone and analyse *bkd* gene, and isolation of *S. toxytricini* bkd-mutants. Additional details of standard techniques, which are well known to those skilled in molecular biology or microbiology, and the designation of the particular enzymes used, are described, for example, in the handbook "Practical Streptomyces genetics" (Kieser et al., 2000, Practical Streptomyces genetics, A laboratory Manual. ISBN0-7084-0623-8).

### Example 1

### Preparation of S. toxytricini Genomic DNA

*Streptomyces toxytricini* CBS 566.68 (wild type) mycelium was grown as a confluent lawn on the Sporulation medium for 8-14 days at 28 °C. The medium comprised:

| | |
|---|---|
| Corn starch | 30 grams |
| Dextrin | 40 grams |
| Soy meal | 30 grams |
| (NH₄)₂SO₄ | 2 grams |
| CaCO₃ | 6 grams |
| Bacteriological agar | 15 grams |
| Final volume | 1 litre |
| pH adjusted to | 7.0 |
| Autoclaved for 35 min at 121°C | |

The grown mycelium was then used to inoculate 50 ml of TSB medium in a 250-ml Erlenmeyer flask, which was maintained with shaking (210 rpm) at 28 °C for 24 hours.

The TSB medium comprised:

| | |
|---|---|
| Peptone from casein | 17.0 grams |
| Peptone from soy meal | 3.0 grams |
| D(+) glucose | 2.5 grams |
| NaCl | 5.0 grams |
| K₂HPO₄ | 2.5 grams |
| Bacteriological agar | 15 grams |
| Final volume | 1 litre |
| pH adjusted to | 7.3 |
| Autoclaved for 20 min at 121°C | |

Cultures were grown for 24 hours at 28 °C. Mycelium was recovered by centrifugation and genomic DNA was prepared following the protocols as described in Kieser et al.(2000, Practical Streptomyces genetics, A laboratory Manual.ISBN0-7084-0623-8). DNA pellets were resuspended in 100 µl TE buffer (Sambrook, and Russell, 2000, Molecular Cloning: A Laboratory Manual, ISBN 978-087969577-4).

### Example 2

### Polymerase Chain Reaction S. toxytricini Genomic DNA Amplification

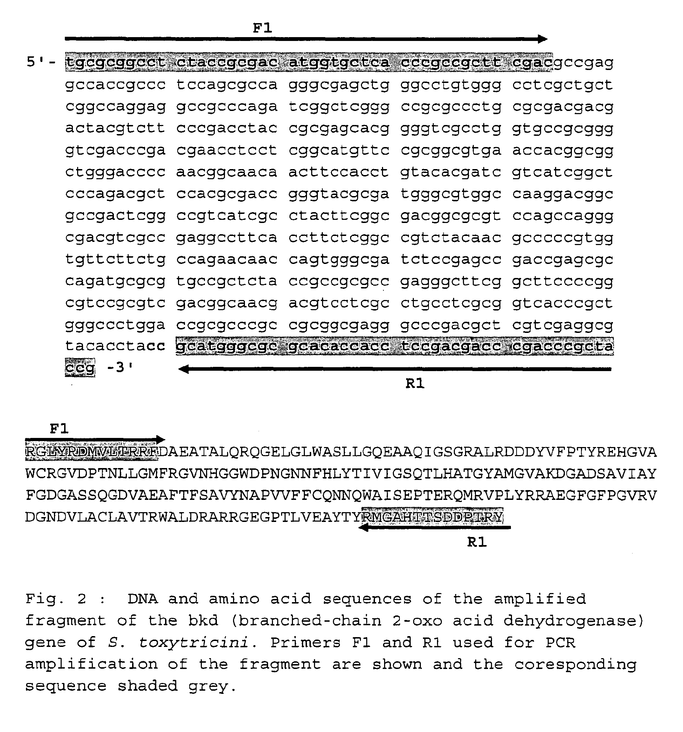

*S. toxytricini* genomic DNA obtained in Example 1 was PCR amplified using a Biorad iCycler Thermal Cycler. The PCR reaction was carried out with Pfu polymerase (New England Biolabs) and the buffer provided by the manufacturer in the presence of 200 µM dNTP, 10% glycerol, 0.5 µM of each primer, approximately 50 ng of template *S. toxytricini* genomic DNA and 2.5 units of enzyme in a final volume of 100 µl for 30 cycles. The thermal profile of all 30 cycles was 95°C for 45 sec (denaturation step), 69°C for 45 sec (annealing step), and 72°C for 1 min (extension step). DNA primers were supplied by Invitrogen. The degenerate primers to amplify a region of bkd gene from *S. toxytricini* are shown in Fig. 2, i.e. a pair binding to highly conserved regions of binding motif (F1) and phosphorylation site (R1) which should yield amplification of approximately 700 bp, and a pair binding to extreme ends of known genes with lower degree of conservation (F0 and R0) which should yield approximately 1000 bp long PCR product. The rightward primer for the amplification of the smaller fragment was bkd-F1 was 5' - TGCGCGGCCTCTACCGSGACATGGTGCTSRCCCGSCGCWTCGAC-3' (SEQ ID NO: 3), and the Leftward primer bkd-R1 was 5'-CGGTASCGGGTSGGGTCGTCGGAGGTGGTGTGCGCGCCCAKSCGG-3' (SEQ ID NO: 4). The amplification products were size fractioned by agarose gel electrophoresis. The PCR sample was electrophoresed in a horizontal 0.8% agarose gel in 1X TBE buffer for 1.5 hours at 92 V as described by Sambrook et al. (2001). The separated PCR DNA products were located in the gel by staining with SybrSafe dye (Invitrogen) and visualizing fluorescent bands with an ultraviolet light. Under the PCR conditions described above, a single DNA band (approximately 700 base pairs long) was detected when using this primer combination. The Rightward primer bkd-F0 was 5'-GAGCTGGTSCAGCTGCTCACCCCCGASGGC-3' (SEQ ID NO: 5), and the Leftward primer bkd-R0 was 5'-GTGCCCKTCSGCGTASACGTKGTCGAASATSKC-3' (SEQ ID NO: 6). The amplification of the larger approximately 1000 bp long PCR fragment bkd0 is done using the same PCR conditions as described for the amplification of the 700 bp fragment.

### Example 3:

Cloning of the 700 bp PCR-amplified *S. toxytricini* DNA Fragment into *E. coli* vector and Subsequent transformation into *E. coli* host.

### A) Recovery of the 700 bp PCR Product

As mentioned before, a 700 bp DNA fragment was amplified by PCR using *S. toxytricini* genomic DNA as template and the Rightward plus Leftward primer combination. The amplified 700 bp DNA fragment was visualized as described before, removed from the gel with a razor blade and the DNA recovered from the agarose by Wizard SV Gel and PCR Clean-Up kit (Promega). The DNA was eluted in 50 µl of water. Isolated fragment was kinased with T4 Polynucleotide Kinase (New England Biolabs) in order to prepare for ligation into a pUC19 *E. coli* vector (Yanisch-Perron et al., 1985 Gene. 33(1):103-19).

### B) SmaI Digestion and Dephosphorylation of Plasmid Vector pUC19

Approximately 1 µg of the plasmid pUC19 (Yanisch-Perron et al., 1985 Gene. 33(1):103-19) was digested with restriction enzyme SmaI (all restriction enzymes were purchased from New England Biolabs) to produce linear blunt-ended molecules. Then, the SmaI-linearized vector was dephosphorylated using Antarctic Phosphatase (New England Biolabs) following the instructions obtained from the supplier. The reaction mixture was incubated for 30 min at 37°C. The vector DNA was "gel-cleaned" by electrophoresis in a horizontal 1.0% agarose gel and eluted as described in Example 2. Final pellet was redissolved in 50 µl of water.

### C) Ligation to produce pUC19-bkd

About 4 µl of the kinased 700 bp PCR DNA product, and about 1 µl of the SmaI-linearized, Antarctic phosphatase-dephosphorylated, blunt-ended pUC19 were incubated overnight with 1 unit of T4 DNA ligase (New England Biolabs) under the conditions specified by the supplier at 10°C in a total reaction volume of 15 µl. The reaction mixture was then used to transform electrocompetent *E. coli* DH10β cells following standard procedure as described by Sambrook et al., 2001. Many white ampicillin resistant transformants were recovered using the blue-white section method with inducer IPTG and substrate X-gal as described in Sambrook et al., 2001. The white colonies should contain the plasmid pUC19-bkd. This was confirmed by selecting several colonies, designated as strains bkd(1-8), and further analyzing. Bacterial colonies of *E. coli* strains bkd(1-8) were inoculated into 2TY liquid medium containing 100 µg/ml of ampicillin following standard microbiological procedures. The cultures were incubated at 37°C overnight. The following morning, the bacterial cells were harvested by centrifugation at 10,000 rpm for 5 min at 4°C. Plasmid vectors were isolated from freshly harvested E. coli bkd(1-8) cells using GenElute Plasmid miniprep kit (Sigma). Isolated plasmids DNA were finally dissolved in 100 µl of water. Confirming restriction analysis, using restriction enzymes EcoRI and XbaI, showed that, as expected, each colony gave a pUC19-bkd plasmid which carried the 700 bp DNA insert.

The 2TY liquid medium comprised:

| | |
|---|---|
| Peptone from casein | 16.0 grams |
| Yeast extract | 10.0 grams |
| NaCl | 5.0 grams |
| Bacteriological agar | 15 grams |
| Final volume | 1 litre |
| pH adjusted to | 7.0 |
| Autoclaved for 20 min at 121 °C | |

### Example 4:

### Sequencing of the cloned 700 bp S. toxytricini genomic fragment

The 700 bp DNA fragment cloned in the plasmid pUC19-bkd was sequenced at Macrogen sequencing facilities (Macrogen Inc, Seoul, Korea) using M13-pUC forward and reverse sequencing primers. DNA sequencing data of the pUC-bkd S. toxytricini genomic fragment is shown in SEQ ID NO: 1.

### Example 5:

### Construction of a temperature-sensitive pKC1139-bkd and suicide pKC1132-bkd plasmids to be used in the disruption of the S. toxytricini bkd gene by homologous recombination

The pUC19-bkd plasmid was digested using EcoRI and HindIII restriction enzymes to isolate the 700 bp DNA fragment of the bkd gene of S. toxytricini. Vectors pKC1139, which contains a normal pUC19-based Ori for replication in E. coli, but a temperature-sensitive Ori for replication in Streptomyces, which is unable to function at elevated temperatures above 35°C (Bierman et al., 1992 Gene. 116(1):43-9, Muth et al., 1989 Mol Gen Genet. 219(3): 341-348), and pKC1132, which only contains the normal pUC19-based Ori for replication in E. coli but cannot maintain replication in Streptomyces (Bierman et al., 1992 Gene. 116(1):43-9), were also digested using EcoRI and HindIII restriction enzymes. Digested vectors were separated using a 1% agarose gel electrophoresis as described in Example 2. Linearized backbone vectors pKC1139 and pKC1132 and the 700 bp fragment of bkd gene were recovered from the agarose gel as described in Example 3. About 4 µl of the EcoRI and HindIII cut 700 bp fragment of bkd gene was added to about 1 µl of the EcoRI and HindIII cut vectors pKC1139 and pKC1132, each, and the mixtures were incubated overnight with 1 unit of ligase (New England Biolabs) under the conditions specified by the supplier at 10°C in a total reaction volume of 15 µl. Each reaction mixture was then used to transform electrocompetent E. coli DH10β cells. Both vectors contain resistance markers for apramycin, so the growth of transformed E. coli DH10β cells was in the 2TY liquid medium with 50 µg/ml apramycin. Isolation of plasmids was as described in Section C, Example 3. Isolated plasmids pKC1139-bkd and pKC1132-bkd were digested with EcoRI and HindIII restriction enzymes to confirm the proper insertion of the 700 bp fragment of bkd gene.

### Example 6:

### Introduction of a frame-shift mutation into the temperature-sensitive pKC1139-bkd and suicide pKC1132-bkd plasmids, creating pKC1139-bkdFS and pKC1132-bkdFS to introduce the frame-shift mutation in the bkd gene of the S. toxytricini genome using secondary homologous recombination

The sequence of the 700 bp fragment of the bkd gene revealed an internal PvuI restriction site CGAT*CG. Vectors pKC1132 and pKC1139 also contain an additional PvuI restriction site, so a partial restriction was made using a serial dilution of the PvuI enzyme in order to obtain the correct restriction internal to the cloned bkd gene fragment. The correct digest was isolated and recovered from the 1% agarose gel as described in Example 2 and Example 3. The cleavage with the PvuI enzyme left a 3' AT overhang, which was blunt-ended with DNA Polymerase I, Large (Klenow) Fragment (New England Biolabs), which contains the 3' to 5' exonuclease activity. The blunt-ended vectors were religated using4 DNA ligase (New England Biolabs), transformed into E. coli DH10β cells, grown and isolated using a plasmid miniprep as described in Section C of Example 3. The removal of the PvuI restriction site by blunt-ending was confirmed by fully digesting the vectors with PvuI, which now generated only linearized vectors pKC1139-bkdFS and pKC1132-bkdFS. Vectors were also sent for sequencing at Macrogen sequencing facilities (Macrogen Inc, Seoul, Korea), confirming the removal of the two base pairs AT from the PvuI restriction site CGATCG, leaving the sequence CGCG with a 2-base pair frame-shift in the sequence.

### Example 7:

### Introduction of a Thiostrepton resistance cassette (Ts) into the temperature-sensitive pKC1139-bkd and suicide pKC1132-bkd plasmids, creating pKC1139-bkdTs and pKC1132-bkdTs to knock-out the bkd gene of the S. toxytricini genome using secondary homologous recombination

Vectors pKC1139-bkd and pKC1132-bkd as obtained in Example 5 were PvuI-digested and blunt-ended as described in Example 6. Blunt-ended vectors were further dephosphorylated by Antarctic Phosphatase to prevent religation. Thiostrepton resistance cassette was obtained from pTS55 (Kieser et al., 2000, Practical Streptomyces genetics, A laboratory Manual. ISBNO-7084-0623-8) by restriction with BamHI and NcoI restriction enzymes, yielding an approximately 900 bp fragment which was blunt-ended with DNA Polymerase I, Large

(Klenow) Fragment. The fragment was isolated by 1% agarose gel electrophoresis and recovered from gel as described in Example 2. Approximately 1 µl of each blunt-ended and dephosphorylated vector was used with approximately 4 µl of the blunt-ended thiostrepton resistance cassette in a 15 µl ligation mix with T4 DNA ligase. Transformation into E. coli DH10β, growth and plasmid isolation was as described in Section C in Example 3. The presence of the thiostrepton resistance cassette in the blunt-ended PvuI restriction site of the bkd gene fragment in pKC1139-bkdTs and pKC1132-bkdTs was confirmed by restriction with EcoRI and HindIII.

### Example 8:

### Introduction of plasmid constructs pKC1139-bkdFS, pKC1132-bkdFS, pKC1139-bkdTs and pKC1132-bkdTs into S. toxytricini by conjugation from E. coli ET12567 containing the conjugative plasmid pUZ8002

Plasmid constructs pKC1139-bkdFS, pKC1132-bkdFS, pKC1139-bkdTs and pKC1132-bkdTs were transformed by transformation into electro competent E. coli strain ET12567 containing the conjugative plasmid pUZ8002 (Paget et al., 1999 J. Bacteriol. 181: 204-211). The plasmid pUZ8002 contains all the necessary genes for construction of conjugative pilli, however it lacks the origin of transfer and, thus, remains in the host cell (Jones et al., 1997 Mol. Microbiol. 23:169-178). Conjugation procedure was done as described in Kieser et al., 2000 (Practical Streptomyces genetics, A laboratory Manual. ISBN0-7084-0623-8). Exconjugants were grown at 28°C on Sporulation medium which is described in Example 1 with addition of 50 µg/ml apramycin. Vectors pKC1132-bkdFS, pKC1139-bkdFS, and pKC1139-bkdTs yielded exconjugants. For the frameshift (FS) approach, only pKC1132-bkdFS exconjugants used further, since exconjugants transformed with pKC1132-based suicide vectors, which do not contain origin of replication for Streptomyces sp., and can only survive on apramycin-containing medium upon successful integration by homologous recombination of the vector into the S. toxytricini genome, thus generating a bkd knock-out strain - a primary recombinant strain. For the bkd gene disruption approach using Thiostrepton resistance conferring cassette, pKC1139-bkdTs exconjugants were used as described in Example 11.

### Example 9:

### Confirmation of bkd-disrupted mutants with integrated pKC1132-based vectors

S. toxytricini primary recombinants were tested for growth on minimal medium supplemented with isoleucine, valine and leucine(NMMB-IVL). The NMMB-IVL medium had been prepared as described below:

| | |
|---|---|
| L-leucine | 2.0 grams |
| L-valine | 2.0 grams |
| L-isoleucine | 2.0 grams |
| (NH₄)₂SO₄ | 1.0 gram |
| K₂HPO₄ | 0.5 gram |
| MgSO₄x7H₂O | 0.2 gram |
| FeSO₄x7H₂O | 0.01 gram |
| K₂HPO₄ | 1.1 grams |
| NaH₂PO₄xH₂O | 1.2 grams |
| Bacteriological agar | 20 grams |
| Final volume | 1 litre |
| Autoclaved for 20 min at 121°C | |

Mutants which were unable to grow on the minimal medium were further tested for branched-chain 2-oxo acid dehydrogenase enzyme activity as described in Example 13. Selected primary recombinant strains were also confirmed by Southern hybridization as described in Example 12. Mutants which did not display any enzyme activity and were unable to grow on the minimal medium were tested for production of lipstatin and determination of impurity profile as described in Example 14. Results are listed in Example 15.

### Example 10:

### Selection of secondary homologous recombination mutants with the frame-shift genotype from which the pKC1132-bkdFS vector removed from the S. toxytricini genome

S. toxytricini primary recombinants with integrated pKC1132-bkdFS vector which had low or zero branched-chain 2-oxo acid dehydrogenase enzyme activity and improved impurity profile are inoculated into liquid TSB medium and grown in shake flasks at 28°C and 210 rpm without the addition of apramycin. Every 24h, 10% of each culture is sub cultivated into a new shake flask with fresh TSB medium. After three consecutive sub cultivations, cultures are plated onto Sporulation medium and grown at 28°C. Harvested spores are filtered and serial dilution is made on plates of Sporulation medium. After 5-8 days single colonies are replica-plated onto plates with Sporulation medium, Sporulation medium with apramycin, and NMMB-IVL minimal medium. Secondary recombinants have lost the apramycin resistance and do not grow on Sporulation medium with apramycin. Revertants to wild type genotype are able to grow successfully on NMMB-IVL minimal medium, while the mutant strains with frame-shift mutation remaining in the bkd gene do not grow, or grow very poorly on the minimal medium. Such mutant strains of S. toxytricini are tested for branched-chain 2-oxo acid dehydrogenase enzyme activity as described in Example 13. Selected secondary recombinant strains are also confirmed by Southern hybridization as described in Example 12. Mutants which do not display any enzyme activity and are unable to grow on the minimal medium are tested for production of lipstatin and determination of impurity profile as described in Example 15.

### Example 11

### Selection of secondary homologous recombination mutants with the bkd gene knock-out by disruption with the thiostrepton resistance cassette from which the pKC1139-bkdTs vector has been removed from the S. toxytricini genome

S. toxytricini exconjugants of the pKC1139-bkdTs, grown at 28°C on Sporulation medium with addition of 50 µg/ml apramycin are inoculated into liquid TSB medium with thiostrepton (25 µg/ml) and grown in shake flasks at 28°C and 210 rpm without the addition of apramycin to produce good seed culture. After 24h, 10% of each seed culture is sub cultivated into a new shake flask with fresh TSB medium with thiostrepton (25 µg/ml) and grown at an increased temperature of 37°C and 210 rpm. At 37°C the pKC1139-based vector is unable to replicate and is forced to integrate into the *S. toxytricini* genome, thus yielding primary recombinants. Every 24h, 10% of each culture is further sub cultivated into a new shake flask with fresh TSB medium with thiostrepton (25 µg/ml). After five consecutive sub cultivations, cultures are plated onto Sporulation medium with thiostrepton and grown at 28°C. Harvested spores are filtered and serial dilution is made on plates of Sporulation medium. After 5-8 days single colonies are replica-plated onto plates with Sporulation medium with thiostrepton, Sporulation medium with thiostrepton and apramycin, and NMMB-IVL minimal medium with thiostrepton. Secondary recombinants have lost the apramycin resistance and do not grow on Sporulation medium with apramycin. Revertants to wild type genotype do not grow on Sporulation medium with thiostrepton, while the mutant strains with bkd knock-out containing the thiostrepton resistance cassette grow well on Sporulation medium with thiostrepton, but do not grow, or grow poorly on minimal medium. Such knock-out mutant strains of S. toxytricini are tested for branched-chain 2-oxo acid dehydrogenase enzyme activity as described in Example 13. Selected secondary recombinant strains are also confirmed by Southern hybridization as described in Example 12. Mutants which do not display any enzyme activity and are unable to grow on the minimal medium are tested for production of lipstatin and determination of impurity profile as described in Example 15.

### Example 12:

### Analysis of S. toxytricini mutants genomic DNA by Southern Hybridization

### A) Preparation of DIG-labelled plasmid DNA probe for pKC1132-bkdFS based mutants

Plasmid DNA of pKC1132-bkdFS was linearized with ClaI restriction enzyme and purified with a Wizard SV Gel and PCR Clean-Up System (Promega). Approximately 1 µg of the linearized plasmid was labelled over-night with digoxigenin-dUTP by random primed labelling technique using the DIG High Prime DNA Labelling and Detection Starter Kit I (Roche).

### B) Preparation of DIG-labelled plasmid DNA probe for pKC1139-bkdTs based mutants

Approximately 1 µg of the plasmid DNA of pKC1139-bkdTs (purified with a Wizard SV Gel and PCR Clean-Up System (Promega)) was labelled over-night with digoxigenin-dUTP by random primed labelling technique using the DIG High Prime DNA Labelling and Detection Starter Kit I (Roche).

### C) DNA transfer

Genomic DNA of S. toxytricini wild type and pKC1132-bkdFS and pKC1139-bkdTs mutants (primary recombinants and secondary recombinants) was prepared using the PureLink Genomic DNA Mini Kit (Invitrogen) according to the instructions of the kit manufacturer. Approximately 10 µg of each isolated genomic DNA was digested with PvuI restriction enzyme. At the end of the digestion, the DNA fragments were separated by electrophoresis in a 1% agarose gel at 20 V for 12 hours, and transferred to a positively charged Hybond-N+ nylon membrane (GE Healthcare, formerly Amersham Biosciences) using the alkali capillary blotting method (Southern, E.M., 1975, J. Mol. BioI., 98:503) for 6 hours. Two separate electrophoreses were run, each transferred separately onto its own Hybond-N+ nylon membrane for each set of the mutants, pKC1132-bkdFS and pKC1139-bkdTs, respectively.

### D) Hybridization

For each set of the mutants, pKC1132-bkdFS and pKC1139-bkdTs, respectively, hybridization was run separately. The same procedure was used both times. Pre-hybridization and hybridization of DIG-labelled DNA probe to DNA immobilized on a nylon membrane was performed as suggested by the DIG High Prime DNA Labelling and Detection Starter Kit I manufacturer (Roche). Pre-hybridization was carried out in the supplied pre-hybridization solution for 1 hour at 50°C. For hybridization, 1 µg of denatured DNA (at 90°C for 10 minutes) was added to fresh pre-hybridization solution pre-warmed to 50°C. After overnight hybridization, membranes were washed as follows: two washes with 2 x SSC, 0.1% SDS, at room temperature for 5 minutes, and two washes with 0.5 x SSC, 0.1% SDS at 55°C for 15 minutes.

### E) Immunological detection

The hybridized probes were immuno-detected with anti-digoxigenin-AP Fab fragments and visualized with the colorimetric substrates NBT/BCIP, as suggested by the DIG High Prime DNA Labelling and Detection Starter Kit I supplier (Roche) .

Primary recombinant of S. toxytricini A676 (CBS122981) with integrated pKC1132-bkdFS vector and secondary recombinant A2072 (CBS122912), bkd disrupted Thiostrepton resistant mutant, derived by pKC1139-bkdTs vector were confirmed by described Southern hybridization procedure.

### Example 13

### Isolation of bkd-disrupted mutants with integrated pKC1132-based vectors, lacking branched-chain 2-oxo acid dehydrogenase enzyme activity

Single colonies were spread as patches onto minimal medium supplemented with leucine, isoleucine and valine(NMMB-IVL) medium and onto Sporulation medium to confirm inability of mutants growth. The plates were incubated for 5-8 days at 28°C. Recombinant mutants which were unable to grow, or grew very poorly, on NMMB-IVL medium were tested on branched-chain 2-oxo acid dehydrogenase enzyme activity in cell extracts.

### Detection of branched-chain 2-oxo acid dehydrogenase enzyme activity by specific assay in crude cell extracts of S. toxytricini mutants

Recombinant mutants which were unable to grow on NMMB-IVL medium were tested on branched-chain 2-oxo acid dehydrogenase enzyme activity in cell extracts. The procedure is described below:

### A) Mycelium preparation

Spores of S. toxytricini from classical and genetic engineered mutants which showed no growth on NMMB IVL medium or grew significantly less on NMMB IVL than wild type were transferred from Sporulation medium patches to 50 ml tubes containing Seed medium supplemented with branched amino acids (e.g. leucine, isoleucine and valine). Seed medium with added branched amino acids was prepared: 10 g soya bean flour, 20 g of glycerol, 5 g of yeast extract, 2 g of L-leucine, 2 g of L isoleucine, 2 g L-valine and water to 1 L. The pH was adjusted to 7.0 with NaOH 10%. 5 ml of this medium was filled into a 50 ml tube, closed with a foam plug and sterilised. Sterilization was performed at 121±2°C, 100+10 kPa for 20 minutes. Shaked tubes were incubated at 28°C for 24 hours.

### B) Cell extracts preparation

1 ml cultures from Seed medium with added branched amino acids were transferred to 1.5 ml tube and cells were collected by centrifugation (5 min at 4500 rpm). Supernatant was removed and pellet was resuspended in 1 ml K-phosphate buffer pH 8.0 (100 mM KH₂PO₄ and 100 mM K₂HPO₄, pH adjusted to 8.0). Cells were centrifuged again and pellet was resuspended in 0.5 ml with K-phosphate buffer. Resuspended cells were sonicated four times for 15 seconds by 100% amplitude with Labsonic M (Sartorius) with 30 seconds pause between two cycles. After sonication 5 µl of 10% Triton X-100 was added and the extracts were placed on ice for 15 minutes. Cell debris was removed by centrifugation for 10 minutes at 14000 rpm. Aliquots of 10 µl of each supernatant were used per enzyme assay. Protein concentration was determined by using Bio-Rad protein assay (Bio-Rad laboratories), which is based on the Bradford dye-binding procedure (Bradford, M., Anal. Biochem., 72:248, 1976).

### C) Assay for branched-chain 2-oxoacid dehydrogenase enzyme activity measurement

The assay for branched-chain 2-oxo acid dehydrogenase was slightly modified method used by Marshall and Sokatch, J. Bact. 110. 1073-1081, 1972. The oxidation of 2-oxoisovalerate and other branched-chain 2-oxo acids was followed by spectrophotometrically measuring the production of reduced nicotinamide adenine dinucleotide (NADH). A reaction volume of 200 microliters contained the cell extract, nicotinamide adenine dinucleotide (NAD+), 1.5 mM; coenzyme A (CoA), 0.13 mM; dithiotreitol (DTT), 3 mM; MgCl₂, 4 mM; thiamine pyrophosphate (TPP), 0.4 mM; K-phosphate buffer, 100 mM, pH 8.0 and L-valine to start enzymatic reaction, 10 mM. Enzyme assays were performed spectrophotometrically (safire² plate reader, Tecan) at 25°C and 340 nm (ε₃₄₀ of reduced pyridine dinucleotide factors, 6.22 mM⁻¹ cm⁻¹) for 10 min with freshly prepared extracts, containing 0.1 to 0.2 mg/ml total proteins.

Primary recombinant mutants S. toxytricini A334 and A708 were so identified and lack branched-chain 2-oxo acid dehydrogenase enzyme activity.

| Strain | Plasmid | BKD spec. activity ^{b,c} |
|---|---|---|
| CBS 566.68 | (w.t.) | 4.0±0.5 |
| A334 | (pKC1139-bkd FS) | 0.0 |
| A654 | (pKC1132-bkd FS) | 0.0 |
| A676 | (pKC1132-bkd FS) | 0.0 |
| A717 | (pKC1132-bkd FS) | 0.0 |
| A2060 | Δbkd Ts ^{a} | 0.0 |
| A2070 | Δbkd Ts ^{a} | 0.0 |
| A2072 | Δbkd Ts ^{a} | 0.0 |
| A2076 | Δbkd Ts ^{a} | 0.0 |

| | | |
|---|---|---|
| ^{a}secondary recombinant mutants with disrupted bkd gene E1-α subunit by integrated thiostrepton cassette. Mutants derived by secondary homologous recombination using pKC1139-bkdTs. ^{b}The specific enzyme activities are reported as U/mg protein, with 1 Unit (U) defined as the production 1 nmol NADH per minute. ^{c}the results are the means of duplicate determinations. | | |

### Example 14

### Fermentative lipstatin production of primary recombinant mutant A676 with pKC1132-bkdFS vector and thiostrepton resistant A2072 bkd disrupted mutant derived by secondary homologous recombination using pKC1139-bkdTs.

### A) Seed culture Process

Seed medium was prepared: 10 g Soya bean flour, 20 g of glycerol, 5 g of yeast extract and water to 1 L. The pH was adjusted to 7.0 with NaOH 10 %. 5 ml of this medium was filled into a 50 ml tube, closed with a foam plug and sterilised. Sterilization was performed at 121±2°C, 100±10 kPa for 20 minutes. The sterilized Seed medium 50 µg/ml apramycin was added and inoculated with a spore suspension of recombinant mutant A676 and incubated on shaker at 28°C, 210 rpm for 20-30 hours under aerobic conditions. Seed culture preparation for thiostreptone resistant A2072 mutant was performed in seed medium without apramycin under the same conditions.

### B) Main Fermentation Process

About 10 % v/v of the above seed culture was used for the inoculation of a 50 ml tube, which contained 5 ml of Fermentation medium. The Fermentation medium contained soy bean flour 32.0 g, glycerol 20.0 g, lecithin 14.0 g, CaCO₃ 2.0 g, MES 2.0 g, soy oil 10.0 ml, linoleic acid 30.0 ml in 1 litre tap water. The pH of the Fermentation medium was adjusted before sterilization to pH 7.40 with 10% NaOH. Sterilization was performed at 121°C for 20 minutes. For mutant A676, after sterilization 50 µg/ml apramycin was added to Fermentation medium on the start of cultivation and after 3 days of cultivation. Fermentation was carried out on shaker at 28°C, 210 rpm for 6-7 days. Fermentation process for thiostreptone resistant mutant A2072 without apramycin addition under the same conditions.

### Example 15

### Determination of lipstatin production and impurity profile determination by LC-MS/MS of primary recombinant mutant A676 with pKC1132-bkdFS vector and thiostrepton resistant A2072 bkd disrupted mutant derived by secondary homologous recombination using pKC1139-bkdTs.

### A) Method for lipstatin production determination:

The analysis for determination of lipstatin or derivatives production thereof was carried out by reversed phase HPLC using an appropriate column and running conditions: column Inertsil C-18 (150x4.0 mm, particle size 3 µm), flow 1.5 ml/min, T°C=45°C, mobile phase: acetonitrile: 0.1% H₃PO₄ =75:25, detection 200 nm.

### B) Methods for lipstatin analogues determination:

The unwanted lipstatin analogues with molecular mass 506 g/mol were determined by LC-MS/MS system (Agilent 1100 series coupled with Watters Micromass Quattro micro detector) using appropriate column and running conditions:
- Column: Gemini 3µ C18 110A (150 x 2 mm) (Phenomenex)
- Column temperature: 45 °C
- Mobile phase: A = 0.05% trifluoroacetic acid - MiliQ water, B = acetonitrile
- Flow: 0.25 ml/min
- Gradient:

| Time (min) | A (%) | B (%) |
|---|---|---|
| 0 | 22 | 78 |
| 13 | 22 | 78 |
| 13.1 | 2 | 98 |
| 23 | 2 | 98 |
| 23.2 | 22 | 78 |
| 30 | 22 | 78 |

- Injection volume: 10 µl of sample + 10 µl of standard
- Detector conditions:
Ionization: ESI⁺
Method: MRM
   1.) Lipstatin analogue with +14 mol. mass: 506→329, 506→347, scan time 2-20 min
   2.) Orlistat: 496→319, 496→337, scan time 2-20 min
Dwell time: 0.1 s
Cone: 20 V
Capillary: 3.0 kV
Extractor: 3 V
Collision energy: 10eV
Source temperature: 120 °C
Desolvation temperature: 350 °C
Cone gas flow: 30 l/h
Desolvation gas flow: 500 l/h
Multiplier: 650 V
Gas Cell Pirani Pressure: 3.0 x 10⁻³ mbar
LM resolution MS1, MS2: 13.0; 12.0
HM resolution MS1, MS2: 13.0; 12.0
- Standard preparation:
1. Weigh 10.0 mg of orlistat into 10 ml flask and bring to volume with methanol (RS1)
2. Take 1.0 ml of RS1 solution into 100 ml flask and bring to volume with methanol (RS2)
3. Take 2.5 ml of RS2 solution into 10 ml flask and bring to volume with methanol (RS) (Standard contains 0.0025 mg orlistat per ml)

- Sample preparation:

1. Take 5 ml of well shaken fermentation broth and centrifuge for 5 min at 4500 rpm
2. Remove supernatant
3. Weigh 1 g of biomass into 50 ml tube and add 5 ml of acetonitrile
4. Put for 1 h on shaker to extract samples
5. Take 1 ml of acetonitrile extract of biomass into 1.5 ml tube and centrifuge for 10 min at 14000 rpm
6. Put 0.8 ml of supernatant into vial and run on LC-MS

The lipstatin analogues content in samples quantification was performed by internal standardization with orlistat and expressed as % of lipstatin-analogue production compared to lipstatin production in samples.

| **Strain** | **Description of strain** | **Lipstatin production (mg/kg)** | **% of lipstatin analogues with mol. mass 506 g/mol compared to lipstatin production** |
|---|---|---|---|
| CBS 566.68 | w.t. | 380 ± 50 | 1.7 |
| A676 | pKC1132-bkd FS | 360 | 0.3 |
| A2072 | Δbkd TS^{a} | 500 | 0.5 |

| | | | |
|---|---|---|---|
| ^{a}Secondary recombinant mutants with disrupted bkd gene E1-α subunit by integrated thiostrepton cassette. Mutants derived by secondary homologous recombination using pKC1139-bkdTs. | | | |

### Example 16

### Production of Branched-chain 2-oxo Acid Dehydrogenase Deficient S. toxytricini with random mutagenesis approach

### A) Step1

Streptomyces toxytricini CBS 566.68 mycelium was grown as a confluent lawn on the Sporulation medium for 8-14 days at 28°C. Spores were harvested from such plate and suspended in 5 ml of 10% glycerol.

### B) Step 2

1 ml of the spore suspension was added to a 1.5 ml Eppendorf tube containing 3 mg of N methyl-N-nitro-N-nitrosoguanidine (NTG). The tube was incubated and shaken at 28°C for 60-180 minutes. Spores were spread on plates with Sporulation medium at different exposure times. Plates were incubated for 8-14 days at 28°C. Spores were harvested from such plate and suspended in 5 ml of 10% glycerol and this suspension was preserved at -20°C and used as a source of cells to be screened for mutants. This spore stock was spread for single colony isolates on Sporulation medium plates approximately 30 colonies per plate. Plates were incubated at 28°C for 5-8 days.

### C) Step 3

After incubation colonies were transferred by replica-plating first onto NMMB-IVL medium and than onto Sporulation medium, for testing their ability to grow in the presence of L-leucine, L-valine, L-isoleucine and combination of said amino acids. The plates were incubated for 5-8 days at 28°C. Then, single colonies from complex sporulation medium which showed no growth or grew significantly less than wild type, were transferred from the Sporulation medium and spread as patches onto minimal medium (NMMB-IVL) and onto Sporulation medium to confirm inability of mutants growth. These patches were than used for testing branched-chain 2-oxo acid dehydrogenase enzyme activity, lipstatin production and determination of lipstatin analogues profile.

### Example 17

Isolation of mutant of S. toxytricini lacking branched-chain 2-oxo acid dehydrogenase enzyme activity using random mutagenesis.

Isolates which were unable to grow or grow very poorly on NMMB-IVL medium were tested on branched-chain 2-oxo acid dehydrogenase enzyme activity in cell extracts as described in Example 13.

Isolated mutants were so identified with decreased or lack of branched-chain 2-oxo acid dehydrogenase enzyme activity.

### Example 18

### Fermentative lipstatin production of isolated mutants from random mutagenesis

### A) Seed culture Process

Seed medium was prepared: 10 g Soy bean flour, 20 g of glycerol, 5 g of yeast extract and water to 1 L. The pH was adjusted to 7.0 with NaOH 10 %. 5 ml of this medium was filled into a 50 ml tube, closed with a foam plug and sterilised. Sterilization was performed at 121±2°C, 100±10 kPa for 20 minutes. The sterilized Seed medium was inoculated with a spore suspension and incubated on shaker at 28°C, 210 rpm for 20-30 hours under aerobic conditions.

### B) Main Fermentation Process

About 10 % v/v of the above seed culture was used for the inoculation of a 50 ml tube, which contained 5 ml of Fermentation medium. The Fermentation medium contained soy bean flour 32.0 g, glycerol 20.0 g, lecithin 14.0 g, CaCO₃ 2.0 g, MES 2.0 g, soy oil 10.0 ml, linoleic acid 30.0 ml in 1 liter tap water. The pH of the Fermentation medium was adjusted before sterilization to pH 7.40 with 10% NaOH. Sterilization was performed at 121°C for 20 minutes. Fermentation was carried out on shaker at 28°C, 210 rpm for 6-7 days.

### Example 19

### Determination of lipstatin production and impurity profile determination v LC-MS/MS of isolated mutants from random mutagenesis

The lipstatin production and lipstatin analogues content in samples quantification was performed by internal standardization with orlistat and expressed as % of lipstatin-analogue production compared to lipstatin production in samples as described in Example 15. Isolated mutants from random mutagenesis showed 50-80% reduction of unwanted lipstatin analogues with molecular mass 506 g/mol.

### Example 20

### Fermentation of lipstatin

Prefermentor / Seed culture medium of following composition was prepared in bioreactor: 350 g of soy bean flour, 350 g of glycerol, 175 g of yeast extract, 70 ml of linoleic acid, 35 ml antifoam agent and tap water to 35 l. The pH was adjusted to 6.5 with 30% H₂SO₄. Sterilisation was performed for 20 minutes at 121°C and subsequently the vessel was cooled to 27°C. Seed culture medium was inoculated with 150 ml of spore suspension. Stirrer speed was set to 200 rpm and the aeration rate was 10 l per minute. In 22 to 30 hours the packed cell volume was more than 12%.

Fermentor / 10 l of this seed culture was used to inoculate a fermentor with a vessel size of 150 l containing 90 l of a production medium containing: 4 kg of soy bean flour, 1 kg of glycerol, 1.5 kg of lecithin, 50 ml of antifoam agent, 200 g of CaCO₃, 1000 g of soy oil, 200 g of linoleic acid, 8 g of FeSO₄, 4 g of ZnSO₄, 12 g of MnCl₂ x 4 H₂O and 12 g of MgSO₄ x 7 H₂O. Before sterilization pH was adjusted to 6.5 with 30% H₂SO₄ and subsequently sterilized for 30 minutes at 121°C. Temperature of fermentation was set to 27°C, stirrer speed was limited to 330 rpm, aeration rate was up to 50 1 per minute. pH was controlled with the addition of 20% NaOH and 30% H₂SO₄ and maintained between 7.1 and 6.8. Within 24 hours concentration of dissolved oxygen dropped to 1%. After reaching that level stirrer speed was regulated to keep the oxygen concentration at about 1%. Feeding with linoleic acid started immediately after the metabolic switch (from glycerol to linoleic acid) twenty hours after inoculation. The rate of feeding of the mixture was controlled by HPLC determination of linoleic acid. Simultaneously with linoleic acid, solution of L-leucine is fed continuously (300g of L- leucine/ 1001 / 160 hours).

### Example 21

### Extraction of lipstatin from fermentation broth

A fermentation broth (360 l) is extracted with ethyl acetate (1080 l). Organic phase is then concentrated in vacuum and finally 10 l ethyl acetate oil (14,7% lipstatin) is obtained. Ethyl acetate oil is further two times extracted with acetonitrile with 12% content of water. For first extraction 40 l and for second 25 l of acetonitrile is used. Acetonitrile extract is concentrated in a vacuum finally 2,5 kg of acetonitrile oil (45,7% lipstatin) is obtained. Water is removed from oil with mixing 6 l of mixture heptan:tbme (70:30). Organic phase is separated and concentrated in vacuum an finally 1,66 kg of oil is obtained

### Example 22

### Extraction of lipstatin from fermentation broth

After the completion of the fermentation process, a volume of 2500 l of fermentation broth (containing 1.9 g/l of lipstatin) was acidified with sulphuric acid (30 %) to the pH value 2.5 ± 0.5. Fermentation broth was charged into a reaction vessel and mixed with 1300 l of ethyl acetate from previous extractions (containing 0.7 g/l of lipstatin) and 700 l of fresh ethyl acetate.
1^{st} Extraction: Mixture was fed into a continuous extractor operated at the feed rate of 2 m³/h. Additional 715 l of fresh ethyl acetate was added as a counter-flow during the extraction. After the completion of extraction 1827 l of ethyl acetate extract was obtained containing 4.0 kg of lipstatin, and 3450 l of extracted broth containing 1.9 kg of lipstatin.
2^{nd} Extraction: 3450 l of extracted broth from the 1^{st} extraction was re-extracted with 1000 l of fresh ethyl acetate. After the completion of the extraction 1383 l of ethyl acetate extract was obtained, containing 1.3 kg of lipstatin and 3040 l of extracted broth containing 0.7 kg of lipstatin.

Ethyl acetate extracts were joined to obtain 5.3 kg of lipstatin in ethyl acetate and further concentrated. Concentrate was re-extracted in acetonitrile twice (volume ratio of concentrate to acetonitrile was 1 : 3) and the undissolved impurities were separated. Acetonitrile phase was further concentrated to obtain crude lipstatin.

### Example 23

### Purification of lipstatin

Silicagel (7,5 g) is mixed with mobile phase
(heptan:TBME=70:30) and chromatography column is filled (height of stationary phase 850 cm, D=15 cm). Acetonitrile oil (830 g) is mixed with mobile phase (1,66 l) and applied to column. Flow of mobile phase is 100 ml/min for first 20 minutes, 180 ml/min for 240 minutes and 270 ml/min till 650 minutes. Fraction (10 l) are collected and analyzed for lipstatin concentration and chrom. purity. Fraction 6-9 were joined and concentrated in vacuum. Lipstatin oil (92,8 %) is obtained appropriate for hydrogenation. HPLC chrom. purity is 95.0%.

### Example 24

### Purification of lipstatin

Silicagel (7,0 g) is mixed with mobile phase
(heptan:TBME=70:30) and chromatography column is filled (height of stationary phase 800 cm, D=15 cm). Acetonitrile oil (830 g) is mixed with silicagel (830 g) and dried in vacuum drier. Dried sample is applied to top of stationary phase in column. Flow of mobile phase is 100 ml/min first 20 minutes, 180 ml/min for 240 minutes and 270 ml/min till 650 minutes. Fraction from 2,5 l to10 l are collected and analyzed for lipstatin concentration and purity. Fraction 10-18 (38liters) were joined and concentrated in vacuum. Lipstatin oil (92,0 %) is obtained appropriate for hydrogenation. HPLC chrom. purity is 95.0%

### Example 25

### Purification of lipstatin

Crude lipstatin was subjected to chromatography purification using silicagel as the stationary phase and mixture t-butyl methyl ether : heptane as a mobile phase.

### Chromatographic conditions

Column: 10 cm x 150 cm filed with 4000 g of silicagel 40 - 63 µm
Mobile phase: t-butyl methyl ether : heptane (volume ratio 40 : 60)
Load: mixture of silicagel and crude lipstatin (weight ration 10 : 1)
Flow rate: 50 ml/min

Fractions containing lipstatin were collected and lipstatin of 92.0 % purity was obtained.

### Example 26

### Purification of lipstatin

Crude lipstatin was subjected to chromatography purification using silicagel as the stationary phase and mixture t-butyl methyl ether : heptane as a mobile phase.

### Chromatographic conditions

Column: 8500 g of silicagel 40 - 63 µm
Mobile phase: t-butyl methyl ether : heptane (volume ratio 40 : 60)
Load: 580 ml of crude lipstatin (HPLC purity 14.8 %)
Flow rate: 30 ml/min

Fractions containing lipstatin were collected and 520 g lipstatin of 82.0 % purity was obtained.

### Example 27

### Purification of lipstatin

Crude lipstatin was subjected to chromatography purification using silicagel as the stationary phase and mixture t-butyl methyl ether : heptane as a mobile phase.

### Chromatographic conditions

Column: 8500 g of silicagel 40 - 63 µm
Mobile phase: t-butyl methyl ether : heptane (volume ratio 40 : 60)
Load: 1700 ml of crude lipstatin (HPLC purity 44.8 %)
Flow rate: 30 ml/min

Fractions containing lipstatin were collected and 630 g lipstatin of 74.0 % purity was obtained.

### Example 28

### Preparation of orlistat by hydrogenation

### Example A

Lipstatin of technical grade (5 g, assay 85.0%), n-heptane (50 ml) and 0,23 g of 5% Pd/C is charged in an autoclave. Autoclave is closed, inertized by nitrogen and then filled by hydrogen to 2 bars. The mixture is stirred at 22 to 27°C and hydrogen pressure is maintained till the consumption of hydrogen is ceased, about 6 hours. Then pressure is released and autoclave is vented by nitrogen. The catalyst is filtered off and the solvent is removed by evaporation in vacuum. 4.42 g of almost colourless oil is obtained. The assay of orlistat is 82.4%, determined by HPLC and no trace of lipstatin is detected.

### Example B

25.3 g of 5% palladium on charcoal (moisture content 58.0%, content of palladium 4.87%) is stirred in ethanol (200 mL), ethanol is filtered off and the cake is washed by n-heptane. The cake is charged in an autoclave and solution of lipstatin, technical grade, (230 g, assay 82.1%) in heptane (2.8 L) is added. Autoclave is closed, inertized by nitrogen and then filled by hydrogen to 10 bar pressure. The mixture is stirred at about 23°C and hydrogen pressure is maintained. When the consumption of hydrogen is ceased, the remaining hydrogen is released and autoclave is vented by nitrogen. The catalyst is filtered off and the solvent is removed by evaporation in vacuum. 205 g of slightly yellowish oil, which during the time converts to a waxy solid, is obtained. Assay of orlistat is 81.2% determined by HPLC.

### Example C

Experiment is performed in the similar way as described under example A, but n-heptane is replaced by n-hexane.

After the catalyst is filtered off and the solvent is removed by evaporation in vacuum, 4.4 g of almost colourless oil is obtained. The assay of orlistat is 83.0%, determined by HPLC and no trace of lipstatin is detected.

### Example D

Experiment is performed in the similar way as described under example A, but n-heptane is replaced by n-nonane.
After the catalyst is filtered off and the solvent is removed by evaporation in vacuum, 4.5 g of almost colourless oil is obtained. The assay of orlistat is 81.3%, determined by HPLC and no trace of lipstatin is detected.

### Example E

Lipstatin (5 g, chrom. purity 95%), n-heptane (50 ml) and 0,55 g of 5% paladium on the charcoal (wet, moisture 58%, just before charging the catalyst is washed by isopropanol and heptane) is charged in an autoclave. Autoclave is closed, inertized by nitrogen and then filled by hydrogen to 20 bars. The mixture is stirred at 22 to 27°C and hydrogen pressure is maintained till the consumption of hydrogen is ceased, about 6 hours. Then the pressure is released and autoclave is vented by nitrogen. The catalyst is filtered off and the solvent is removed by evaporation in vacuum. 4.8 g of almost colourless oil is obtained. The assay of orlistat is 89.2%, determined by HPLC. HPLC chrom. purity is 96%.

### Example F

Lipstatin (173 g, chrom. purity 95%), n-heptane (3000 ml) and 19.7 g of 5% paladium on the charcoal (wet, moisture 58%, just before charging the catalyst is washed by ethanol and heptane) is charged in an autoclave. Autoclave is closed, inertized by nitrogen and then filled by hydrogen to 10 bars. The mixture is stirred at 22 to 32°C and the hydrogen pressure is maintained till the consumption of hydrogen is ceased, about 8 hours. Then the pressure is released and autoclave is vented by nitrogen. The catalyst is filtered off and the solvent is removed by evaporation in vacuum. 156.0 g of almost colourless oil is obtained. The assay of orlistat is 90.2%, determined by HPLC. HPLC chrom. purity is 95.2%.

### Example 29

### Preparation of orlistat by hydrogenation

Orlistat can be synthesized by hydrogenation of lipstatin as obtained after extraction or purification step.

Lipstatin, (35 g), ethanol (0,8 L) and 3,3 3 g of 5% Pd/C was charged in a reactor and stirred for a few hours at 10 to 40°C. Then the mixture is filtered and the clear solution was charged to an autoclave vessel and additional 3,3 g of 5% palladium on active carbon was added. Autoclave was closed, inertised by nitrogen and filled by hydrogen to 3.5 bar. The mixture was stirred at 15 to 25°C till the consumption of hydrogen was ceased. Then hydrogen is released and autoclave is vented by nitrogen. The catalyst is filtered off and the solvent is evaporated off. 34,7 g of slightly yellowish oil, which during the time converts to a waxy solid, was obtained.

### Example 30

### Crystallization of orlistat

Orlistat, as prepared in previous example - hydrogenation/Example E - (4.6 g) is charged in a flask, dissolved in n-heptane (14 ml) at about 30°C. The solution is cooled while stirred to 20°C, seeded by a few milligrams of orlistat form II and cooled down slowly (in 2 about hours) to 15°C. The suspension is stirred for additional few hours at 15°C and the solid product is filtered off. The assay of orlistat is 80%, chromatografic purity (HPLC) is 98.6% and no trace of lipstatin is detected. Polymorphic form of product corresponds to form II, as described in the patent application WO 2007/073937.

### Example 31

### Crystallization of orlistat

Orlistat obtained by hydrogenation (15 kg, assay 97,6%), a mixture of the form I and form II as described in WO 2005/026140, and heptane (60 L) are charged in an glass lined reactor and stirred by impeller stirrer. Suspension is dissolved by heating and then it is cooled to about 22°C, seeded by 10 g of orlistat form II, stirred at the same temperature for 1 hour and cooled to 15°C in about 2 hours. The obtained crystals are aged for 15 hours and filtered off by suction. After drying in vacuum, 14,1 kg of material is obtained. The XRPD of this material corresponds to orlistat form II, assay 100,4% using HPLC method.

### Example 32

### Formulation containing orlistat prepared by granulation

### Example A

| Ingredient | mg/capsule |
|---|---|
| Orlistat | 120 |
| Cellulose, microcrystalline | 150 |
| Total | 270 |

1. Orlistat and cellulose, microcrystalline (Microcel MC-200) are sieved, mixed in high share mixer (225.6 g of mixture) and granulated with the ethanol as granulating liquid.
2. Granules are dried at or below 25°C (input air 28-30 °C) and passed through 18 Mesh screen. The loss on drying (measurement performed by Mettler Tolledo HR73 halogen moisture analyzer at 85 °C for 20 minutes) of the obtained granules was 1.17 % by weight
3. Additional cellulose, microcrystalline (of the same type) is added (44.4 g) to the granulate and mixed. The mixture was dried additionally. The loss on drying 0.17 % by weight was obtained.
4. Mixture was filled in a hydroxypropyl methyl cellulose (HPMC) and gelatine based capsules.

One part of the capsules was packed into Alu-Alu blister packs and the other part of the capsules was not packed.

In stress stability testing the amount of hydrolytic impurities has not changed in closed containers (Alu-Alu blister packs) at 30°C/65 % RH nor in opened containers weeks at the same conditions.

High resolution HPLC is used to determine orlistat and related impurities (area %). The tests are carried out in Zorbax XDB C18, 50 X 4,6 mm column (particle size 1,8 pm) . The mobile phase is acetonitrile ACN : 0,1% acetic acid solution = 83 : 17 (Vol./Vol.). The chromatograph is equipped with UV detector.

The term hydrolytic impurity refers hereby to hydrolytic orlistat degradation products, such as (2S,3R,5S)-5-[(S)-2-formylamino-4-methyl-pentanoyloxy]-hexyl-3-hydroxy-hexadecanoic acid (H1); (2S,3S,5S)-5-[(N-formyl-L-leucyl)oxy]-2-hexyl-3-hydroxyhexadecanoic acid (H2), N-formyl-L-leucine (3S,4R,6S)-tetrahydro-3-hexyl-2-oxo-6-undecyl-2H-pyran-4-yl ester (H3).

| Impurity | t0 | 30°C/65%RH - opened container, granulate | |
|---|---|---|---|
| | | 1 month | 3 months |
| H1 | blq (0.023) | blq (0.028) | blq (0.046) |
| H2 | bld | bld | bld |
| H3 | bld | bld | bld |
| Decarboxyl degradation product | bld | bld | bld |

| Impurity | t0 | 30°C/65%RH - AluAlu blisters | 30°C/65%RH - PVC/PE/PV DC blisters | 30°C/65%RH - opened container, capsule mixture | |
|---|---|---|---|---|---|
| | | 1 month | 1 month | 4 weeks | 12 weeks |
| H1 | bld | blq (0.038) | blq (0.042) | blq (0.037) | blq (0.050) |
| H2 | bld | bld | bld | bld | bld |
| H3 | bld | blq (0.054) | blq (0.052) | blq (0.051) | blq (0.057) |
| Decarboxyl degradation product | bld | bld | bld | bld | bld |

| Impurity | t0 | 30°C/65%RH - AluAlu blisters, HPMC capsules | | 30°C/65%RH - PVC/PVDC listers, gelatine capsules | | 30°C/65%RH - granulate, opened | |
|---|---|---|---|---|---|---|---|
| | | 1 month | 4 months | 1 month | 4 months | 1 month | 4 months |
| H1 | bld | blq (0.02 0) | blq (0.05 0) | blq (0.02 1) | blq (0.05 2) | blq (0.019 ) | blq (0.03 8) |
| H2 | bld | bld | bld | bld | bld | bld | bld |
| H3 | bld | bld | bld | bld | bld | bld | bld |
| Decarboxyl degradation product | bld | bld | bld | bld | bld | bld | bld |

| Impurity | t0 | 30°C/65%RH - opened container, granulate | |
|---|---|---|---|
| | | 1 month | 4 months |
| H1 | bld | blq (0.020) | blq (0.040) |
| H2 | bld | bld | bld |
| H3 | bld | bld | bld |
| Decarboxyl degradation product | bld | bld | bld |

| | | | |
|---|---|---|---|
| bld: bellow the limit of detection dlq: bellow the limit of quantification ( ≤ 0.1%) | | | |

### Example B

Example A was repeated with the exception that the cellulose, microcrystalline of types Microcel MC-102 and MC 250 were used.

SEQUENCE LISTING

<110> Krka, d.d.,

<120> Process for production of lipstatin and microorganisms therefore

<130> 126873

<160> 6

<170> PatentIn version 3.1

<210> 1
<211> 703
<212> DNA
<213> Streptomyces toxytricini

<400> 1
tgcgcggcct ctaccgcgac atggtgctca cccgccgctt cgacgccgag gccaccgccc 60
tccagcgcca gggcgagctg ggcctgtggg cctcgctgct cggccaggag gccgcccaga 120
tcggctcggg ccgcgccctg cgcgacgacg actacgtctt cccgacctac cgcgagcacg 180
gggtcgcctg gtgccgcggg gtcgacccga cgaacctcct cggcatgttc cgcggcgtga 240
accacggcgg ctgggacccc aacggcaaca acttccacct gtacacgatc gtcatcggct 300
cccagacgct ccacgcgacc gggtacgcga tgggcgtggc caaggacggc gccgactcgg 360
ccgtcatcgc ctacttcggc gacggcgcgt ccagccaggg cgacgtcgcc gaggccttca 420
ccttctcggc cgtctacaac gcccccgtgg tgttcttctg ccagaacaac cagtgggcga 480
tctccgagcc gaccgagcgc cagatgcgcg tgccgctcta ccgccgcgcc gagggcttcg 540
gcttccccgg cgtccgcgtc gacggcaacg acgtcctcgc ctgcctcgcg gtcacccgct 600
gggccctgga ccgcgcccgc cgcggcgagg gcccgacgct cgtcgaggcg tacacctacc 660
gcatgggcgc gcacaccacc tccgacgacc cgacccgcta ccg 703

<210> 2
<211> 233
<212> PRT
<213> Streptomyces toxytricini

<400>  2

Arg Gly Leu Tyr Arg Asp Met Val Leu Thr Arg Arg Phe Asp Ala Glu
1               5                   10                  15

Ala Thr Ala Leu Gln Arg Gln Gly Glu Leu Gly Leu Trp Ala Ser Leu
            20                  25                  30

Leu Gly Gln Glu Ala Ala Gln Ile Gly Ser Gly Arg Ala Leu Arg Asp
        35                  40                  45

Asp Asp Tyr Val Phe Pro Thr Tyr Arg Glu His Gly Val Ala Trp Cys
    50                  55                  60

Arg Gly Val Asp Pro Thr Asn Leu Leu Gly Met Phe Arg Gly Val Asn
65                  70                  75                  80

His Gly Gly Trp Asp Pro Asn Gly Asn Asn Phe His Leu Tyr Thr Ile
                85                  90                  95

Val Ile Gly Ser Gln Thr Leu His Ala Thr Gly Tyr Ala Met Gly Val
            100                 105                 110

Ala Lys Asp Gly Ala Asp Ser Ala Val Ile Ala Tyr Phe Gly Asp Gly
        115                 120                 125

Ala Ser Ser Gln Gly Asp Val Ala Glu Ala Phe Thr Phe Ser Ala Val
    130                 135                 140

Tyr Asn Ala Pro Val Val Phe Phe Cys Gln Asn Asn Gln Trp Ala Ile
145                 150                 155                 160

Ser Glu Pro Thr Glu Arg Gln Met Arg Val Pro Leu Tyr Arg Arg Ala
                165                 170                 175

Glu Gly Phe Gly Phe Pro Gly Val Arg Val Asp Gly Asn Asp Val Leu
            180                 185                 190

Ala Cys Leu Ala Val Thr Arg Trp Ala Leu Asp Arg Ala Arg Arg Gly
        195                 200                 205

Glu Gly Pro Thr Leu Val Glu Ala Tyr Thr Tyr Arg Met Gly Ala His
    210                 215                 220

Thr Thr Ser Asp Asp Pro Thr Arg Tyr
225                 230

<210>  3

<211>  44

<212>  DNA

<213>  artificial sequence

<220>
<223>  primer bkd-F1

<400>  3
tgcgcggcct ctaccgsgac atggtgctsr cccgscgcwt cgac                    44

<210>  4
<211>  45
<212>  DNA
<213>  artificial sequence

<220>
<223>  primer bkd-R1

<400>  4
cggtascggg tsgggtcgtc ggaggtggtg tgcgcgccca kscgg                   45

<210>  5
<211>  30
<212>  DNA
<213>  artificial sequence

<220>
<223>  primer bkd-F0

<400>  5
gagctggtsc agctgctcac ccccgasggc                                    30

<210>  6
<211>  33
<212>  DNA
<213>  artificial sequence

<220>
<223>  primer bkd-R0

<400>  6
gtgcccktcs gcgtasacgt kgtcgaasat skc                                33

## Claims

1. Microorganism capable of producing lipstatin, **characterized by** being unable to grow on a minimal medium comprising valine, leucine and isoleucine as sole carbon source, preferably **characterized by** a reduced or abolished activity of at least one functional subunit of the branched-chain 2-oxo acid dehydrogenase multi-enzyme complex.

2. The microorganism according to claim 1, wherein the activity of at least one gene coding for the E1 alpha, E1 beta, E2 and E3 subunits of the branched-chain 2-oxo acid dehydrogenase multi-enzyme complex or one or more regulatory sequences promoting the activity of one or more of these genes has been reduced or abolished, preferably wherein the activity of the E1 alpha gene coding for branched-chain 2-oxo acid dehydrogenase and/or of a regulatory sequence promoting activity thereof has been reduced or abolished.

3. The microorganism according to claim 1 or 2, **characterized by** a specific activity of branched-chain 2-oxo acid dehydrogenase below 1.5 U/mg total protein, preferably 0 U/mg total protein, as determined in a cell free extract, wherein 1 U represents the generation of 1 nmol NADH per minute.

4. The microorganism according to any one of claims 1 to 3 which belongs to Actinomycetes, preferably belongs to the genus Streptomyces, more preferably is selected from the group consisting of Streptomyces toxytricini and Streptomyces virginiae.

5. Nucleotide sequence suitable for reducing or abolishing, in a microorganism capable of producing lipstatin, the activity of at least one functional subunit of branched-chain 2-oxo acid dehydrogenase multi-enzyme complex, preferably suitable for reducing or abolishing the specific activity of branched-chain 2-oxo acid dehydrogenase to less than 1.5 U/mg total protein as determined in a cell free extract, wherein 1 U represents the generation of 1 nmol NADH per minute,
preferably selected from a mutated nucleotide sequence derived from a sequence coding for branched-chain 2-oxo acid dehydrogenase, and a mutated promoter sequence derived from a promoter of branched-chain 2-oxo acid dehydrogenase.

6. Nucleotide sequence encoding the E1 alpha subunit of the branched-chain 2-oxo acid dehydrogenase of an organism belonging to the genus Streptomyces, comprising the sequence according to SEQ ID NO: 1.

7. Nucleotide sequence according to claim 6, comprising
a) one or more nucleotide additions, deletions, substitutions or inversions;
b) the sequence according to a) having at least 80% identity, preferably at least 90% identity with SEQ ID NO: 1;
c) a nucleic acid sequence capable of hybridizing under stringent conditions to the a nucleotide sequence, or its complement, according to a) or b), or to SEQ ID NO: 1;
wherein said sequence according to a), b), or c) has the capacity to encode the E1 alpha subunit of the branched-chain 2-oxo acid dehydrogenase of an organism belonging to the genus Streptomyces.

8. Process for obtaining a microorganism according to any one of claims 1 to 4, comprising mutating a microorganism and selecting a mutant having decreased or abolished activity of at least one gene coding for the four subunits of the branched-chain 2-oxo acid dehydrogenase multi-enzyme complex.

9. The process according to claim 8 which comprises mutating a nucleotide sequence according to claims 6 or 7, or using a sequence according to claim 5.

10. Process for the manufacture of lipstatin comprising the use of the microorganism according to any one of claims 1 to 4 or the microorganism obtainable by the process according to claim 8 or 9.

11. Process for the manufacture of orlistat, comprising manufacture of lipstatin according to the process of claim 10, and hydrogenation of lipstatin to obtain orlistat.

12. Use of a microorganism according to any one of claims 1 to 4, a microorganism obtainable by the process according to claim 8 or 9, or a nucleotide sequence according to claim 5 in a process for producing lipstatin and/or orlistat.

13. Lipstatin obtainable by the process according to claim 10.

14. Orlistat obtainable by the process according to claim 11.

15. The lipstatin according to claim 13 or orlistat according to claim 14, **characterized by** a content of branched chain analogues below 5% by weight.

16. Process for the manufacture of a medicament comprising orlistat, comprising use of a microorganism according to any one of claims 1 to 4, a nucleotide sequence according to claim 5, lipstatin according to claim 14 or orlistat according to claim 15.
